# EUROPEAN PATENT APPLICATION

(11) **EP 2 100 874 A1**
(43) Date of publication of application: **16.09.2009**
(21) Application number: 07831211.3
(22) Date of filing: 05.11.2007
(51) Int. Cl.: C07C 209/36, C07C 209/86, C07C 211/52, C07B 61/00

(54) **PROCESS FOR PRODUCTION OF 2,2'-BIS(TRIFLUOROMETHYL)- 4,4'-DIAMINOBIPHENYL**

(30) Priority: 13.11.2006 JP 2006306312
(71) Applicant: Toray Fine Chemicals Co., Ltd., Urayasu-shi, Chiba 279-8555 (JP)
(72) Inventor: NAKATANI, Jiro, Moriyama-shi Shiga 524-0041 (JP)
(74) Representative: Kador & Partner
(86) International application number: PCT/JP2007/071478
(87) International publication number: WO 2008/059724

(57) **Abstract**

The present invention is a method for producing of 2,2'-bis(trifluoromethyl)-4,4'-diaminobiphenyl, wherein 2,2'-bis(trifluoromethyl)biphenyl is produced from o-chlorobenzotrifluoride, further, 2,2'-bis(trifluoromethyl)biphenyl is dinitrated in 1,2-dichloropropane solution, and 2,2'-bis(trifluoromethyl)-4,4'-dinitrobiphenyl is isolated and reduced. The method for producing of 2,2'-bis(trifluoromethyl)-4,4'-diaminobiphenyl of the present invention is an industrially excellent method for producing with high safety and high production efficiency.

## Description

### Technical Field

The present invention relates to a method for producing of 2,2'-bis(trifluoromethyl)-4,4'-diaminobiphenyl.

### Background Art

2,2'-Bis(trifluoromethyl)-4,4'-diaminobiphenyl is a compound widely used in a polymer chemistry field. In particular, 2,2'-bis(trifluoromethyl)-4,4'-diaminobiphenyl is useful as a raw material of polyimide, polyamide and so on. Polyimide and polyamide including 2,2'-bis(trifluoromethyl)-4,4'-diaminobiphenyl have been used as industrial applications in various fields such as electronic information materials and optical materials.

As a method for producing of 2,2'-bis(trifluoromethyl)-4,4'-diaminobiphenyl, using 2-haloganated benzotrifluoride as a raw material, known is a method for producing including:
(1) a process of producing 2,2'-bis(trifluoromethyl)biphenyl by coupling it in the presence of metal, and isolating the 2,2'-bis(trifluoromethyl)biphenyl by a distillation method (coupling process);
(2) a process of dinitrating 2,2'-bis(trifluoromethyl)biphenyl with a mixed acid of sulfuric acid and nitric acid to produce 2,2'-bis(trifluoromethyl)-4,4'-dinitrobiphenyl, and isolating by a crystallization method (dinitration process); and
(3) a process of reducing 2,2'-bis(trifluoromethyl)-4,4'-dinitrobiphenyl to produce 2,2'-bis(trifluoromethyl)-4,4'-diaminobiphenyl, and isolating by crystallization (reduction process).

In the coupling process (1), a method that copper is used as a reactant for 2-iodobenzotrifluoride is proposed (see Patent Document 1). However, 2-iodobenzotrifluoride is expensive, and 2,2'-bis (trifluoromethyl) biphenyl produced is expensive. Further, resulting from using a lot of copper, there arise environmental problems due to waste liquid and waste material treatments etc. On the other hand, in Non-patent document 1, an expensive nickel catalyst is used for 2-chlorobenzotrifluoride, and zinc is used as a reactant. However, resulting from using a lot of zinc, there are environmental problems due to waste liquid and waste material treatments etc (see Non-Patent Document 1).

In Non-Patent Document 1, in the dinitration process (2), 2,2'-bis(trifluoromethyl)biphenyl undergoes nitration reaction without solvents. However, since reaction temperature is high in a method described in Non-Patent Document 1, position selectivity of dinitration to 4,4' position of biphenyl backbone is low (see Non-Patent Document 1). Since the method described in Non-Patent Document 1 produces many isomers, the load becomes large for isolating 2,2'-bis(trifluoromethyl)-4,4'-dinitrobiphenyl. Further, because the viscosity of reaction liquid is extremely high, it is very difficult to isolate a target material from the reaction liquid industrially.

On the other hand, in a method described in Patent Document 1, reaction is progressed at low temperature by using methylene chloride as a solvent and, a solvent and a mixed acid as a nitration agent are separated in two layers, thus it was easy to isolate 2,2'-bis(trifluoromethyl)-4,4'-dinitrobiphenyl (see Patent Document 1). However, methylene chloride is a substance designated as group 2B (possible carcinogen to human) in carcinogenic evaluations by International Agency for Research on Cancer (IARC), and there are problems in environments and operations. Further, methylene chloride has been registered in Europe as a banned substance.

In Patent Document 2, in the reduction process (3), reduction is carried out by using hydrazine in the presence of a palladium catalyst (see Patent document 2). However, hydrazine has high toxicity and is an explosive hazardous substance, so there were problems in operations.

Non-Patent Document 1 describes a method that reduction is conducted with ammonium formate in the presence of a palladium catalyst (see Non-Patent Document 1). However, since a plurality of solvents are used in large amounts, substrate concentration was low and production efficiency was bad.

Patent Document 3 describes a method that catalytic reduction is conducted in an alcoholic solvent with hydrogen in the presence of a palladium catalyst. Although this method has high production efficiency, coloring of reaction liquid is notable, so a purification operation is necessary to remove this coloring (see Patent Document 3).
[Patent document 1] Japanese Patent Application Laid-open No. 3- 44355
[Patent document 2] Japanese Patent Application Laid-open No.2006-56786
[Patent document 3] US Patent No. 2004/0013337 Publication
[Non-patent document 1] Journal of polymer Society Part A, Polymer Chemistry, vol. 31, p. 2081 to 1091 (1993).

### Disclosure of the Invention

The present invention is a method for producing of 2,2'-bis(trifluoromethyl)-4,4'-diaminobiphenyl, comprising:
(1) a first process of producing 2,2'-bis(trifluoromethyl)biphenyl by coupling o-chlorobenzotrifluoride, and isolating 2,2'-bis(trifluoromethyl)biphenyl;
(2) a second process of dinitrating 2,2'-bis(trifluoromethyl)biphenyl in 1,2-dichloropropan solution, and isolating 2,2'-bis(trifluoromethyl)-4,4'-dinitrobiphenyl by crystallization; and
(3) a third process of reducing 2,2'-bis(trifluoromethyl)-4,4'-dinitrobiphenyl, and isolating 2,2'-bis(trifluoromethyl)-4,4'-diaminobiphenyl.

### Best Mode for Carrying Out the Invention

The first process in the method for producing of 2,2'-bis(trifluoromethyl)-4,4'-diaminobiphenyl of the present invention is that after 2,2'-bis(trifluoromethyl)biphenyl is produced by subjecting o-chlorobenzotrifluoride to coupling reaction, followed by isolation.

The chemical formula of 2,2'-bis(trifluoromethyl)-4,4'-diaminobiphenyl is shown below.

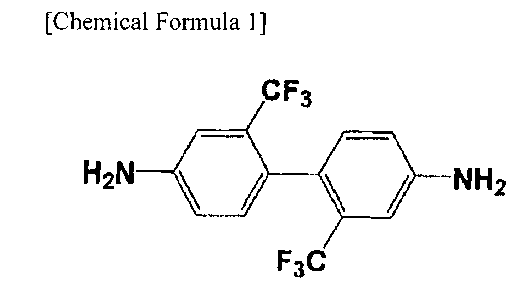

The chemical formula of 2.2'-bis(trifluoromethyl)biphenyl is shown below.

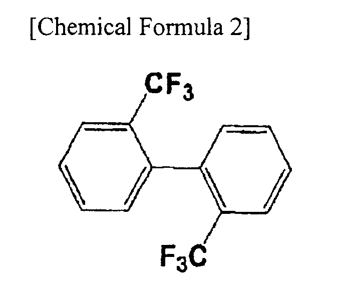

As a method for coupling o-chlorobenzotrifluoride, the present invention preferably uses a method that a chlorine atom of o-chlorobenzotrifluoride is reacted with magnesium metal to convert into a Grignard reagent, and Grignard reagents are coupled. The conversion reaction into a Grignard reagent can utilize a known conversion reaction.

In the reaction of a chlorine atom of o-chlorobenzotrifluoride with magnesium metal, it is preferable to use powdery magnesium metal. The conversion reaction into a Grignard reagent is carried out in a dehydrated system. In the conversion reaction into a Grignard reagent, it is preferable to use a dehydrated solvent. In the conversion reaction into a Grignard reagent, it is preferable to add an inexpensive Grignard reagent and remove water.

In the conversion reaction into a Grignard reagent, in order to remove a surface oxide layer of the magnesium metal for enhancing reactivity, it is preferable to add iodine, bromine or a compound containing these. As examples of iodine, bromine or a compound containing these, there are preferably listed methyl iodide, methyl bromide, ethyl iodide, ethyl bromide and the like.

In the method for producing of the present invention, it is preferable to use a catalyst in the coupling reaction. As a preferable catalyst, there is listed at least one metal selected from Fe, Ag, Cu, Co, Zn, Ni and Pd, or compound thereof. As a preferable compound, there are exemplified chloride, bromide, iodide, fluoride, acetate, acetylacetonate, carbonate, hydroxide and nitrate of these metals. As a catalyst, ferrous chloride (II), ferric chloride (III), ferrous bromide and ferric bromide are particularly preferable.

The amount of catalyst used is preferably 0.01% by mole to 20% by mole relative to 1 mole of o-chlorobenzotrifluoride, and further preferably 0.05% by mole to 10% by mole. By setting the use amount of catalyst to 0.01 % by mole to 20% by mole relative to 1 mole of o-chlorobenzotrifluoride, coupling reaction can be carried out efficiently and economically.

In the method for producing of the present invention, it is preferable to conduct the coupling reaction under the coexistence of an oxidant. The oxidant oxidizes a catalyst reduced by the coupling reaction for regeneration, therefore, an index showing how much reaction products can be produced by one molecule of a catalyst, Turnover Number increases and reaction yield improves.

As the oxidant, from the viewpoints of handleability and separation from products, a halogenated aliphatic hydrocarbon is preferable, in particular, a halogenated aliphatic hydrocarbon with carbon numbers of 1 to 5 is preferable. Preferable halogenated aliphatic hydrocarbons with carbon numbers of 1 to 5 include chloroethane, dichloroethane, trichloroethane, tetrachloroethane, tetrachloroethylene. pentachloroethane, hexachloroethane, bromoethane, dibromoethane, tribromoethane. tetrabromoethane, chloropropane, dichloropropane, trichloropropane, chlorobutane, dichlorobutane, chloropentane, dichloropentane, bromopropane, dibromopropane, tribromopropane, bromochloromethane and bromochloroethane. Among these, preferable are dichloromethane, chloroethane, dichloroethane, dichloropropane, dibromomethane, bromoethane, dibromoethane and dibromopropane.

In the first process of the method for producing of 2,2'-bis(trifluoromethyl)-4,4'-diaminobiphenyl of the present invention, the oxidant is particularly preferably 1,2-dichloroethane or 1,2-dichloropropane.

The amount of oxidant used is preferably 0.1 mole times to 5 mole times relative to 1 mole of o-chlorobenzotrifluoride, and is 0.2 mole times to 3 mole times. When the use amount of oxidant is 0.1 mole times to 5 mole times relative to 1 mole of o-chlorobenzotrifluoride, an effect of catalyst regeneration by an oxidant is large, no unreacted oxidant is left, and no load arises in isolation and purification of a target material.

The solvent used in the first process in the method for producing of the present invention is preferably a solvent capable of efficiently progressing the reaction. The solvent used in the first process in the method for producing of the present invention is preferably an ether-type solvent easily producing a Grignard reagent. As a specific example of the preferable solvent, there are listed diethyl ether, diisopropyl ether, tetrahydrofuran, N,N-dimethylformamide, N,N-dimethylacetamide, N-methylpyrrolidone, 1,3-dioxane, 1,4-dioxane, cyclopropyl methyl ether, methyl tert-butyl ether, monoglyme, diglyme, triglyme, tetraglyme, benzene, toluene, xylene, and the like. Above all, preferable solvents are diethyl ether, diisopropyl ether, tetrahydrofuran, 1,3-dioxane. 1.4-dioxane, cyclopropyl methyl ether and methyl tert-butyl ether.

The use amount of solvent is preferably 0.5 to 100 mole times relative to o-chlorobenzotrifluoride. When the use amount of solvent is 0.5 to 100 mole times relative to o-chlorobenzotrifluoride, the yield of a Grignard reagent is good, leading to an economical process.

In the method for producing of the present invention, the reaction temperature of coupling reaction is preferably 30 to 100°C, and further preferably 45 to 70°C. When the reaction temperature of coupling reaction is 30 to 100°C, the reaction proceeds without any problems.

In the present invention, in the coupling reaction, chloro-2,2'-bis(trifluoromethyl)biphenyl is produced as a byproduct. When 2.,2'-bis(trifluoromethyl)biphenyl is used without separation and removal of chloro-2,2'-bis(trifluoromethyl)biphenyl produced as a byproduct as raw materials such as fine chemicals, pharmaceutical and agrichemical raw materials, resin and plastic raw materials, electric information materials and optical materials, there arise problems in quality such as deterioration of qualities of final products, that is, purity deterioration, coloring, strength deterioration, deterioration of optical characteristics. Therefore, the present invention isolates 2,2'-bis(trifluoromethyl)biphenyl. In the method for producing of the present invention, preferably in the first process, a reaction liquid after coupling reaction is poured into water or an acidic aqueous solution, after mixing, the mixture is allowed to stand still and separated in two layers, then from an oil layer separated and obtained, 2,2'-bis(trifluoromethyl)biphenyl is distilled for isolation.

In the method for producing of the present invention, a method that 2,2'-bis(trifluoromethyl)biphenyl is isolated and obtained from the coupling reaction liquid by a distillation method is preferably used.

Further preferably, there is used a method that before distillation, the coupling reaction liquid is poured into water or an acidic aqueous solution, the remaining Grignard reagent, active magnesium or the like is inactivated, and after magnesium salts are removed to an aqueous layer, distillation for isolation from the resulting oil layer separated is carried out.

As the distillation method, simple distillation, rectification, vacuum distillation and atmospheric distillation are listed, and vacuum distillation is preferably used. It is preferable to lower chloro-2,2'-bis(trifluoromethyl)biphenyl produced as a byproduct in reaction as little as possible. Since chloro-2,2'-bis(trifluoromethyl)biphenyl has a higher boiling point than 2,2'-bis(trifluoromethyl)biphenyl, there is preferably used a distillation operation that 2,2'-bis(trifluoromethyl)biphenyl is distilled away while distilling away chloro-2,2'-bis(trifluoromethyl)biphenyl as little as possible, being left in the bottom.

In the method for producing of the present invention, the content of chloro-2,2'-bis(trifluoromethyl)biphenyl in the 2,2'-bis(trifluoromethyl)biphenyl obtained is preferably 0.01% by weight to 20% by weight, and further preferably 0.01 % by weight to 5% by weight. By setting the content of chloro-2,2'-bis(trifluoromethyl)biphenyl in 2,2'-bis(trifluoromethyl)biphenyl to 0.01 % by weight to 20% by weight, qualities concerning purity, coloring, strength, optical characteristics and the like of 2,2'-bis(trifluoromethyl)-4,4'-diaminobiphenyl derived from 2.2'-bis(trifluoromethyl)biphenyl, and polymers synthesized therefrom are maintained.

In the second process of the method for producing of the present invention, 2,2'-bis(trifluoromethyl)biphenyl is dinitrated in 1,2-dichloropropane solution, and 2,2'-bis(trifluoromethyl)-4,4'-dinitrobiphenyl is isolated by crystallization.

The chemical formula of 2,2'-bis(trifluoromethyl)-4,4'-dinitrobiphenyl is shown bellow.

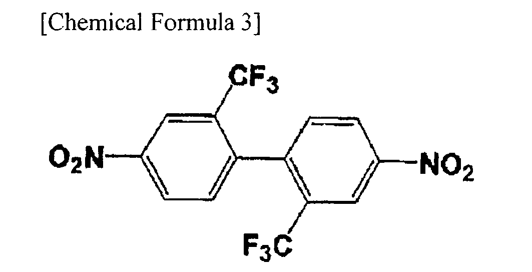

In the nitration reaction in the second process of the method for producing of the present invention, 1,2-dichlorpropane is used as a solvent. 1,2-Dichlorpropane is classified as group 3 (substance not classifiable as to carcinogenicity to human) in carcinogenic evaluations by International Agency for Research on Cancer (IARC), and is less toxic than methylene chloride conventionally used as a nitration solvent, thus problems in environments and operations are reduced.

The amount of 1,2-dichlorpropane used is preferably 0.5 mole times to 100 mole times relative to 1 mole of 2,2'-bis(trifluoromethyl)biphenyl, and is 3 mole times to 20 mole times. When less than 0.5 mole times, there is a case that an effect of solvent becomes small, and that an efficient dinitration reaction hardly proceeds. When more than 100 mole times, there is a case that production efficiency becomes bad.

A nitration agent in the method for producing of the present invention preferably uses a mixed acid of sulfuric acid and nitric acid in general. The mixed acid is ordinarily sulfuric acid of 1 to 5 mole ratios relative to nitric acid of 1 mole, and water content in the mixed acid is not more than 10% by weight, preferably not more than 5% by weight, and further preferably not more than 3% by weight. The use amount is preferably 2 mole ratios to 5 mole ratios as nitric acid relative to 2,2'-bis(trifluoromethyl)biphenyl.

In the nitration reaction in the second process of the method for producing of the present invention, a preferable range of reaction temperature is 10°C to 100°C, further preferably 30°C to 70°C.

As the method for taking out 2,2'-bis(trifluoromethyl)-4,4'-dinitrobiphenyl from a reaction liquid for example, a method that the reaction liquid is allowed to stand still before it is separated into an oil layer and a mixed acid layer, then separated, after the oil layer obtained is subjected to alkali washing and/or water washing, 1,2-dichloropropane as a solvent is concentrated, and 2,2'-bis(trifluoromethyl)-4,4'-dinitrobiphenyl is obtained by crystallization is preferably used. Further, to obtain high-purity one, recrystallization may be done using an alcoholic solvent, a halogen-type solvent, or a petroleum solvent.

In the third process of the method for producing of the present invention, 2,2'-bis(trifluoromethyl)-4,4'-dinitrobiphenyl is reduced, and 2,2'-bis(trifluoromethyl)-4,4'-diaminobiphenyl is isolated.

In the third process of the method for producing of the present invention, preferably, 2.2'-bis(trifluoromethyl)-4,4'-dinitrobiphenyl is catalytically reduced with hydrogen in the presence of a metal catalyst, and 2,2'-bis(trifluoromethyl)-4,4'-diaminobiphenyl is isolated by crystallization.

In the reduction process in the third process of the method for producing of the present invention, a method by using molecular hydrogen in the presence of a metal catalyst is preferably used.

As a metal catalyst used, an ordinary reduction catalyst can be used. As the metal catalyst, for example, it is possible to use nickel, palladium, platinum, rhodium, ruthenium, cobalt, copper and the like. Industrially, it is preferable to use a palladium or platinum catalyst as a metal catalyst. These metal catalysts can be used in a metal state. Further, these metal catalysts are ordinarily used, from the advantageous points of separation, collection and reuse, with being supported on the substrate surface of carbon, barium sulfate, silica gel, alumina, sellite and the like. These metal catalysts can be also used as Raney catalysts such as nickel, cobalt and copper.

The amount of catalyst used in the third process of the method for producing of the present invention is preferably 0.001 to 1% by weight in terms of metal relative to 2,2'-bis(trifluoromethyl)-4,4'-dinitrobiphenyl of the raw material, and more preferably 0.05 to 0.5% by weight.

In the third process of the method for producing of the present invention, a reaction solvent is preferably one inactive to the reaction. As the reaction solvent, for example, there can be used alcohols such as methanol, ethanol and isopropyl alcohol; glycols such as ethylene glycol and propylene glycol; ethers such as dioxane, tetrahydrofuran and methyl cellosolve; aliphatic hydrocarbons such as hexane and cyclohexane; aromatic hydrocarbons such as benzene, toluene and xylene; esters such as ethyl acetate and butyl acetate; halogenated hydrocarbons such as dichloromethane, chloroform, carbon tetrachloride, 1,2-dichloroethane and tetrachloroethane; N,N-dimethylformamide and the like.

In the third process of the method for producing of the present invention, in the case that an aromatic hydrocarbon and/or an aliphatic alcohol with carbon numbers of not less than 2 are used as a solvent for reduction reaction, it is preferable because the resulting 2,2'-bis(trifluoromethyl)-4,4'-diaminobiphenyl has little coloring, and toluene, xylene, isopropanol and normal propanol are further preferable.

In the third process of the method for producing of the present invention, the use amount of solvent is sufficient in an amount just for a raw material to be suspended or dissolved completely. In the third process of the method for producing of the present invention, the use amount of solvent is ordinarily 1.0 to 20 by weight relative to 2,2'-bis(trifluoromethyl)-4,4'-dinitrobiphenyl.

In the third process of the method for producing of the present invention, the reaction temperature is generally 5 to 100°C, preferably 20 to 80°C.

In the third process of the method for producing of the present invention, hydrogen pressure in the reaction is preferably 0.1 to 15 MPa, further preferably 0.3 to 2 MPa. When the hydrogen pressure is 0.1 to 15 MPa, the reaction is fast and economical.

In the method for producing of the present invention, as a method for isolating 2,2'-bis(trifluoromethyl)-4,4'-diaminobiphenyl from the reaction liquid containing 2,2'-bis(trifluoromethyl)-4,4'-diaminobiphenyl obtained by reduction, isolation of 2,2'-bis(trifluoromethyl)-4,4'-diaminobiphenyl by a crystallization method is preferably used.

As the crystallization method, for example, there is a method that after separation of the catalyst and reaction liquid by filtration, the reaction solvent is distilled away and concentrated to separate out crystals, and solid-liquid separation is conducted; or a method that an acidic aqueous solution is added to a reaction liquid to form salts, which are completely dissolved in water, then after impurities insoluble in water are filtered away, the filtrate is neutralized with alkali to separate out 2,2'-bis(trifluoromethyl)-4,4'-diaminobiphenyl, and solid-liquid separation is conducted, and either method may be suitable, further, for higher purification and lower coloring, the crystal obtained may be dissolved again in an organic solvent for recrystallization.

As a recrystallization solvent, for example, there can be used alcohols such as methanol, ethanol and isopropyl alcohol; glycols such as ethylene glycol and propylene glycol; ethers such as dioxane, tetrahydrofuran and methyl cellosolve; aliphatic hydrocarbons such as hexane and cyclohexane; aromatic hydrocarbons such as benzene, toluene and xylene; esters such as ethyl acetate and butyl acetate; halogenated hydrocarbons such as dichloromethane, chloroform, carbon tetrachloride, 1,2-dichloroethane and tetrachloroethane; N,N-dimethylfomiamide and the like, in particular, in the case that an aliphatic hydrocarbon or an aromatic hydrocarbon is used, it is preferable because the resulting 2,2'-bis(trifluoromethyl)-4,4'-diaminobiphenyl has little coloring, and an aromatic hydrocarbon is further preferable.
The 2,2'-bis(trifluoromethyl)-4,4'-diaminobiphenyl obtained by the method for producing of the present invention has been used as industrial applications in various fields such as electronic information materials and optical materials, and it is valuable that the compound can be obtained industrially at low cost and high efficiency.

### [Examples]

Hereinafter, the present invention is described further in detail by Examples.
Additionally, maker-grades of reagents used here all correspond to a first class or higher.

### [Example 1]

Tetrahydrofuran of 143.6 g (1.99 mol; manufactured by nacalai tesque Inc.) and magnesium power of 16. 1 g (0.664 mol; manufactured by Chuo-Kosan Co., Ltd.) were charged in a reactor equipped with a thermometer, and stirred while replacing the system with nitrogen. Tert-butyl magnesium chloride of 2 g (0.017 mol; manufactured by Tokyo Chemical Industry Co., Ltd.) was added thereto, and moisture in the system was removed. Next, o-chlorobenzotrifluoride of 10 g (0.0554 mol; manufactured by MITENI Corp.) was charged, subsequently ethyl bromide of 2 g (0.018 mol; manufactured by nacalai tesque Inc.) was added. In stirring for a while, heat generation was confirmed. Next, while keeping the temperature of reaction liquid at 35 to 50°C, o-chlorobenzotrifluoride of 90 g (0.499 mol) was added dropwise. After completion of dropping, the liquid was aged while stirring at 45°C for 3 hours. The yield of Grignard reagent was 91%.

Next, to a catalyst-containing solution of 1,2-dichloropropane of 75.0 g (0.664 mol; manufactured by Wako Pure Chemical Industries Ltd.) added to a liquid obtained by adding tetrahydrofuran of 54 g (0.72 mol) to ferric chloride (III) of 2.70 g (0.0166 mol; manufactured by Wako Pure Chemical Industries Ltd.), the above-described Grignard reagent solution was added dropwise while keeping the temperature of reaction liquid at 45 to 60°C. After completion of dropping, the reaction was carried out at 65°C for 3 hours. The yield of 2,2'-bis(trifluoromethyl)biphenyl relative to o-chlorobenzotrifluoride was 73%. Chloro-2,2'-bis(trifluoromethyl)biphenyl produced as a byproduct was 1.7% by weight relative to 2,2'-bis(trifluoromethyl)biphenyl.
The above-described reaction liquid was poured in a 3% hydrochloric acid aqueous solution of 400 g put in a 1000 ml separating funnel, mixed well for 30 minutes at room temperature, and allowed to stand still for 30 minutes. After still standing, the mixture was separated, thereby obtaining an oil layer. Then, simple distillation of this oil layer was conducted under reduced pressure. After foreshot was cut away, distillate of 48.1 g at 100 to 130°C under a condition of 1.33 kPa in vacuum was obtained. The concentration of 2,2'-bis(trifluoromethyl)biphenyl in the distillate obtained was 97.1 % by weight, and the concentration of chloro-2,2'-bis(trifluoromethyl)biphenyl was 0.5% by weight.

This 2,2'-bis(trifluoromethyl)biphenyl of 42.0 g and 1,2-dichloropropane of 136.8 g were charged in a 300 ml reactor equipped with a thermometer, and dissolved. A mixed acid of 66.9 g obtained by mixing 97% nitric acid of 13.7 g and 97% sulfuric acid of 53.2 g was added thereto dropwise while cooling so that reaction temperature became 5 to 10°C, and stirred for 0.5 hours. Subsequently, a mixed acid of 66.9 g obtained by mixing 97% nitric acid of 13.7 g and 97% sulfuric acid of 53.2 g was added dropwise at 25 to 30°C, and then, stirred at 35°C for 10 hours. The conversion of 2,2'-bis(trifluoromethyl)biphenyl was 100%, and the selectivity of 2,2'-bis(trifluoromethyl)-4,4'-dinitrobiphenyl was 95.5%. A mixed acid layer was separated, and an oil layer obtained was washed twice with 50 g of water. 1,2-Dichloropropane in this oil layer was distilled away under reduced pressure, concentrated and crystallized, thereby obtaining 2,2'-bis(trifluoromethyl)-4,4'-dinitrobiphenyl crystal of 42.5 g (purity-converted) by solid-liquid separation.

This 2,2'-bis(tritluoromethyl)-4,4'-dinitrobiphenyl crystal of 42.5 g, toluene of 127.5 g and 5% Pd/C (AER-TYPE: 50% water-contained product) manufactured by N.E. CHEMCAT Corp. were fed in an autoclave made of stainless steel, after the system was replaced sufficiently with hydrogen, pressured with hydrogen for a reaction pressure to be 1 MPa, and reaction was carried out at a reaction temperate of 60°C for 6 hours.

After reaction, catalyst was filtered away, toluene in this reaction liquid was distilled away under reduced pressure, concentrated and crystallized, 2,2'-bis(trifluoromethyl)-4,4'-diaminobiphenyl of 28.5 g (purity-converted) was obtained by solid-liquid separation.

### Industrial Applicability

The method for producing of 2,2'-bis(trifluoromethyl)-4,4'-diaminobiphenyl of the present invention uses an inexpensive aromatic chloride as a starting base material, and by using 1,2-dichloropropane with low toxicity, it becomes an industrially excellent method for producing with high safety and high production efficiency.

## Claims

1. A method for producing of 2,2'-bis(trifluoromethyl)-4,4'-diaminobiphenyl, comprising:
(1) a first process of producing 2,2'-bis(trifluoromethyl)biphenyl by coupling o-chlorobenzotrifluoride and isolating 2,2'-bis(trifluoromethyl)biphenyl;
(2) a second process of dinitrating 2,2'-bis(trifluoromethyl)biphenyl in 1,2-dichloropropan solution, and isolating 2,2'-bis(trifluoromethyl)-4,4'-dinitrobiphenyl by crystallization; and
(3) a third process of reducing 2,2'-bis(trifluoromethyl)-4,4'-dinitrobiphenyl, and isolating 2,2'-bis(trifluoromethyl)-4,4'-diaminobiphenyl.

2. The method for producing of 2,2'-bis(trifluoromethyl)-4,4'-diaminobiphenyl according to claim 1,
wherein in the first process, a chlorine atom of o-chlorobenzotrifluoride is reacted with magnesium metal to convert into a Grignard reagent, and the Grignard reagents are coupled in the presence of a catalyst and an oxidant.

3. The method for producing of 2,2'-bis(trifluoromethyl)-4,4'-diaminobiphenyl according to claim 2,
wherein in the first process, the oxidant is a halogenated aliphatic hydrocarbon.

4. The method for producing of 2,2'-bis(trifluoromethyl)-4,4'-diaminobiphenyl according to claim 3,
wherein in the first process, the halogenated aliphatic hydrocarbon is 1,2-dichloroethane or 1,2-dichloropropane.

5. The method for producing of 2,2'-bis(trifluoromethyl)-4,4'-diaminobiphenyl according to claim 2,
wherein in the first process, the catalyst is at least one metal selected from Fe, Ag, Cu, Co, Zn, Ni and Pd, or compound thereof.

6. The method for producing of 2,2'-bis(trifluoromethyl)-4,4'-diaminobiphenyl according to claim 1,
wherein in the first process, a reaction liquid after the coupling reaction is poured into water or an acidic aqueous solution, after mixing, the mixture is allowed to stand still and separated in two layers, then from an oil layer separated and obtained, 2,2'-bis(trifluoromethyl)biphenyl is distilled for isolation.

7. The method for producing of 2,2'-bis(trifluoromethyl)-4,4'-diaminobiphenyl according to claim 1,
wherein in the second process, the reaction temperature is 10°C to 100°C.

8. The method for producing of 2,2'-bis(trifluoromethyl)-4,4'-diaminobiphenyl according to claim 1,
wherein in the third process, 2,2'-bis(trifluoromethyl)-4,4'-dinitrobiphenyl is catalytically reduced with hydrogen in the presence of a metal catalyst, and 2,2'-bis(trifluoromethyl)-4,4'-diaminobiphenyl is isolated by crystallization.

9. The method for producing of 2,2'-bis(trifluoromethyl)-4,4'-diaminobiphenyl according to claim 8,
wherein in the third process, an aromatic hydrocarbon and/or an aliphatic alcohol with carbon numbers of not less than 2 are used as a solvent of the reduction reaction.
